Europäisches Patentamt

European Patent Office

Office européen des brevets

(19)

(11) Veröffentlichungsnummer: **0 073 945**
**B1**

(12) **EUROPÄISCHE PATENTSCHRIFT**

(45) Veröffentlichungstag der Patentschrift:
08.05.85

(21) Anmeldenummer: **82107282.4**

(22) Anmeldetag: **11.08.82**

(51) Int. Cl.⁴: **C 07 C 7/10,** C 07 C 15/02,
C 07 C 7/08

(54) Verfahren zur Gewinnung reiner Aromaten.

<table>
<tr><td>

(30) Priorität: **05.09.81 DE 3135319**

(43) Veröffentlichungstag der Anmeldung:
**16.03.83 Patentblatt 83/11**

(45) Bekanntmachung des Hinweises auf die Patenterteilung:
**08.05.85 Patentblatt 85/19**

(84) Benannte Vertragsstaaten:
**AT BE DE FR GB IT NL**

(56) Entgegenhaltungen:
**AT - B - 308 716**
**US - A - 3 639 497**

**Die Akte enthält technische Angaben, die nach dem Eingang der Anmeldung eingereicht wurden und die nicht in dieser Patentschrift enthalten sind.**

</td><td>

(73) Patentinhaber: **Krupp-Koppers GmbH, Moltkestrasse 29, D-4300 Essen 1 (DE)**

(72) Erfinder: **Preusser, Gerhard, Dr., Dipl.-Chem., Lönsberg 24, D-4300 Essen 1 (DE)**
Erfinder: **Schulze, Martin, Krüdenscheider Weg 83, D-5604 Neviges (DE)**
Erfinder: **Emmrich, Gerd, Dipl.-Ing., Kamperfeld 21, D-4300 Essen 1 (DE)**
Erfinder: **Schneider, Hans-Christoph, Rosental 77, D-4320 Hattingen (DE)**

</td></tr>
</table>

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Verfahren zur Gewinnung reiner Aromaten aus Kohlenwasserstoffgemischen, die neben diesen Aromaten einen beliebig hohen Gehalt an Nichtaromaten enthalten, durch Extraktivdestillation mit N-substituierten Morpholinen als selektivem Lösungsmittel, wobei der Extraktionsstufe eine Vordestillation zugeordnet ist.

Die Abtrennung von Aromaten aus Kohlenwasserstoffgemischen durch Flüssig-Flüssig-Extraktion und/oder Extraktivdestillation mit N-substituierten Morpholinen, mit oder ohne Wasserzusatz, als selektivem Lösungsmittel ist bereits bekannt. So werden beispielsweise in der DE-PS 1 568 940 und der AT-PS 308 716 zwei derartige Verfahren beschrieben, die sich in der Praxis bereits im großtechnischen Maßstab bewährt haben. Hierbei ist es vielfach üblich, insbesondere, wenn die Extraktivdestillation zur Anwendung gelangt, daß das als Einsatzprodukt verwendete Kohlenwasserstoffgemisch vor dem Eintritt in die Extraktionsstufe einer Vordestillation unterworfen wird. Die Extraktionsstufe besteht dabei normalerweise aus der oder den Extraktivdestillationskolonnen sowie der dazugehörigen Abtriebskolonne, in der das Lösungsmittel destillativ von den als Extrakt gewonnenen Aromaten abgetrennt wird. Die Vordestillation dient hierbei dazu, bestimmte Bestandteile aus dem Einsatzprodukt vor dem Eintritt in die Extraktionsstufe abzutrennen. Sofern das Einsatzprodukt mehrere Aromaten, z. B. Benzol, Toluol und Xylole enthält, muß der vom Lösungsmittel befreite Extrakt in einer der Extraktionsstufe nachgeschalteten Destillation (Nachdestillation) in die genannten Bestandteile weiterzerlegt werden.

Aus der US-PS 3 639 497 ist dabei ein Aromatengewinnungsverfahren bekannt, bei dem die als Flüssig-Flüssig-Extraktion betriebene Extraktionsstufe in einem Wärmeverbund mit den nachgeschalteten Fraktionierkolonnen zur Aufarbeitung des aromatenhaltigen Extraktes betrieben wird. Dieser Wärmeverbund erfolgt in der Weise, daß der Druck, unter dem die nachgeschalteten Fraktionierkolonnen betrieben werden, so erhöht wird, daß die Temperatur der dort anfallenden Kopfproduktdämpfe so hoch ist, daß dieselben zur Kolonnenbeheizung in der Extraktionsstufe genutzt werden können.

Obwohl, wie bereits gesagt wurde, die beiden zuerst genannten Patentschriften im großtechnischen Maßstab brauchbare Verfahren zur Aromatengewinnung beschreiben, ist man natürlich auch auf diesem Arbeitsgebiet bestrebt, den Energieverbrauch derartiger Verfahren noch weiter herabzusetzen. Der Erfindung liegt deshalb die Aufgabe zu Grunde, das Verfahren der eingangs erwähnten Art dahingehend zu verbessern, daß bei der Durchführung desselben eine deutliche Energieeinsparung erzielt werden kann.

Das der Lösung dieser Aufgabe dienende Verfahren ist erfindungsgemäß dadurch gekennzeichnet, daß die Vordestillation bei erhöhtem Druck bis zu 20 bar und bei Temperaturen bis zu 300° C durchgeführt wird, wobei der Druck jeweils so eingestellt wird, daß die Vordestillation bei höherer Temperatur betrieben werden kann als die Extraktionsstufe und der Wärmeinhalt der dabei anfallenden Dämpfe zur Kolonnenbeheizung in der bei niedriger Temperatur betriebenen Extraktionsstufe genutzt wird.

Das erfindungsgemäße Verfahren sieht also einen Wärmeverbund zwischen der als Extraktivdestillation betriebenen Extraktionsstufe und der Vordestillation in der Weise vor, daß die Vordestillation unter Anwendung erhöhter Drücke auf einem Temperaturniveau betrieben wird, das eine Nutzung des Wärmeinhaltes der dabei anfallenden Dämpfe zur Beheizung der Kolonnen in der Extraktionsstufe gestattet. Gegenüber einer bei Normaldruck betriebenen Vordestillation lassen sich hierbei, bezogen auf das Gesamtverfahren, Energieeinsparungen erzielen, die mehr als 40% betragen können.

Im Hinblick auf die Tatsache, daß es sich bei den als Einsatzprodukt für die Aromatengewinnung verwendeten Kohlenwasserstoffgemischen in der Regel um kompliziert zusammengesetzte Vielstoffgemische handelt, wird man die erfindungsgemäße Arbeitsweise auch nicht als naheliegend ansehen können. Gegenüber der Normaldruckdestillation verschieben sich nämlich bei derartigen Vielstoffgemischen die Destillationsverhältnisse (Azeotrop-Bildung, Siedepunkte etc.) bei Anwendung von erhöhten Drücken in einer Weise, die sich auf Grund von theoretischen Berechnungen nicht eindeutig vorhersagen läßt. Es ist außerdem davon auszugehen, daß bei einer unter erhöhtem Druck betriebenen Destillation höhere Betriebs- und Anlagekosten anfallen als bei einer Normaldruckdestillation. Dies äußert sich z. B. in wesentlich höheren Rückflußverhältnissen, erhöhter Bodenzahl sowie einer aufwendigeren Kolonnenkonstruktion. Faßt man die vorstehend aufgeführten Kriterien zusammen, so spricht zunächst nichts dafür, zum Zwecke der Energieeinsparung, bei der Vordestillation die Arbeitsweise unter normalem Druck durch eine Arbeitsweise unter erhöhtem Druck zu ersetzen. Die beim erfindungsgemäßen Verfahren erzielte Energieeinsparung war deshalb durchaus überraschend.

Das erfindungsgemäße Verfahren wird in der Regel so durchgeführt, daß die Vordestillation unter erhöhtem Druck bis zu 20 bar betrieben wird, während in der Extraktionsstufe bei Normaldruck oder unter mäßig erhöhtem Druck bis maximal 3 bar gearbeitet wird. In der Extraktionsstufe kann dabei eine ein- oder mehrstufige Extraktivdestillation zur Anwendung gelangen. Es kann aber auch eine Kombination von Extraktivdestillation und Flüssig-Flüssig-Extraktion eingesetzt werden.

Die in der Vordestillation anfallenden Dämpfe können nicht nur zur Beheizung der Extraktivdestillationskolonnen sowie der dazugehörigen Abtriebskolonnen genutzt werden. Ein Teil dieser Dämpfe kann auch im indirekten Wärmeaustausch zur Vorwärmung des Einsatzproduktes herangezogen werden.

Das in der Vordestillation einzuhaltende Rückflußverhältnis wird dabei im Sinne der Erfindung nicht allein — wie sonst üblich — durch die zu bewältigende Trennaufgabe bestimmt. Das Rückflußverhältnis und die Bodenzahl werden in der Vordestillationskolonne vielmehr so gewählt, daß die dabei am Kopf der Kolonne anfallende Dämpfemenge für die Kolonnenbeheizung in der Extraktionsstufe sowie die gegebenenfalls erforderliche Vorwärmung des Einsatzproduktes ausreicht.

Als Einsatzprodukte für das erfindungsgemäße Verfahren kommen vorzugsweise Kokereibenzol-Druckraffinat, hydriertes Pyrolysebenzin, Reformatbenzin sowie andere, Benzol, Toluol und Xylole enthaltende Kohlenwasserstoffgemische in Frage.

Wenn die Vordestillation unter erhöhtem Druck betrieben wird, können die dabei anfallenden Dämpfe unter Umständen einen höheren Gehalt an bestimmten Nichtaromaten, wie z. B. Methylcydohexan, aufweisen. Da jedoch diese Dämpfe nach ihrer Kondensation das Einsatzprodukt für die nachgeschaltete Extraktionsstufe darstellen, könnten sich hieraus Probleme ergeben, weil diese Nichtaromaten bei Anwendung bestimmter Lösungsmittel in der Extraktionsstufe nur sehr schwer von den Aromaten abzutrennen sind. Diese Probleme werden beim erfindungsgemäßen Verfahren jedoch dadurch vermieden, daß dasselbe an die Verwendung von N-substituierten Morpholinen als selektives Lösungsmittel gebunden ist. Hierbei hat sich insbesondere das N-Formylmorpholin als geeignetes Lösungsmittel erwiesen. Die vorstehend genannten Lösungsmittel können dabei mit oder ohne Wasserzusatz eingesetzt werden. Sofern ein Wasserzusatz zum Lösungsmittel vorgesehen ist, liegt dieser normalerweise bei der Extraktivdestillation zwischen 0 und 8 Gew.-%.

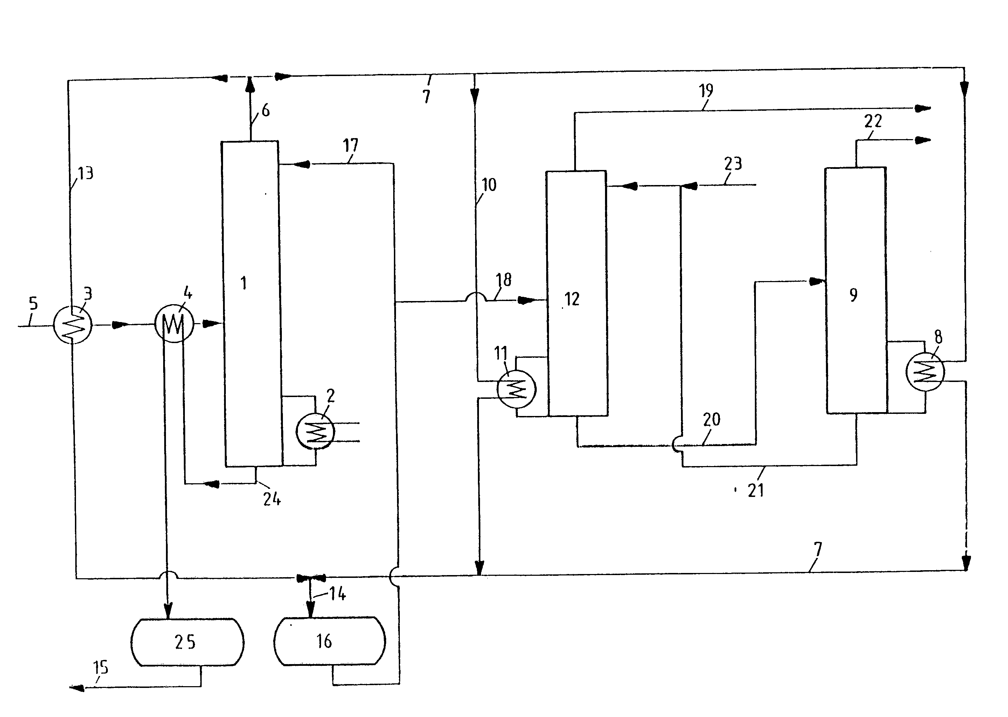

Weitere Einzelheiten des erfindungsgemäßen Verfahrens ergeben sich aus den vorliegenden Unteransprüchen sowie der nachfolgenden Verfahrenserläuterung an Hand des in der Abbildung dargestellten Fließ-Schemas.

Das dargestellte Fließ-Schema zeigt nur die für die Verfahrenserläuterung unbedingt notwendigen Einrichtungen, während sonstige Nebeneinrichtungen, wie zum Beispiel Ventile und Pumpen, nicht dargestellt sind. Das als Einsatzprodukt dienende aromatenhaltige Kohlenwasserstoffgemisch wird über die Leitung 5 und die Wärmeaustauscher 3 und 4 in den mittleren Teil der mit Böden versehenen und unter erhöhtem Druck betriebenen Vordestillationskolonne 1 eingeleitet. Am Fuße dieser Kolonne befindet sich der Sumpfumlaufkocher 2, dessen Röhrensystem mit Hochdruckdampf oder über einen nicht dargestellten Ofen beheizt werden kann. Das dampfförmige Kopfprodukt der Vordestillation wird über die Leitung 6 am Kopf der Vordestillationskolonne 1 abgezogen. Im vorliegenden Fall wird der Dampfstrom in der Leitung 6 in zwei Teilströme aufgeteilt. Der größere Teilstrom gelangt hierbei über die Leitung 7 in das Röhrensystem des Sumpfumlaufkochers 8, in dem die fühlbare Wärme der Dämpfe zur Beheizung der Abtriebskolonne 9 genutzt wird. Gleichzeitig wird ein Teilstrom der Dämpfe aus der Leitung 7 über die Leitung 10 abgezeigt und durch das Röhrensystem des Sumpfumlaufkochers 11 geleitet, der der Beheizung der Extraktivdestillationskolonne 12 dient.

Gleichzeitig gelangt außerdem der kleinere Teilstrom der Kopfproduktdämpfe aus der Leitung 6 über die Leitung 13 in das Röhrensystem des indirekten Wärmeaustauschers 3, in dem die fühlbare Wärme der Dämpfe zur Vorwärmung des Einsatzproduktes genutzt wird. Die hinter dem Wärmeaustauscher 3 und den Sumpfumlaufkochern 8 und 11 anfallenden Kondensate gelangen, wie im Fließ-Schema dargestellt wird, zur Sammelleitung 14, die zum Sammelbehälter 16 führt. In diesem wird das kondensierte Kopfprodukt aus der Vordestillationskolonne 1 gesammelt. Über die Leitung 17 wird dasselbe aus dem Sammelbehälter 16 abgezogen. Die Leitung 17 führt zum Kopf der Vordestillationskolonne 1, so daß ein kleiner Teil des Kopfproduktes als Rücklauf wieder auf die Vordestillationskolonne 1 aufgegeben werden kann. Die Hauptmenge desselben wird jedoch über die Leitung 18 als Einsatzprodukt in den mittleren Teil der mit Böden versehenen Extraktivdestillationskolonne 12 eingeleitet.

In der Extraktionsstufe, d. h. in der Extraktivdestillationskolonne 12 und der dazugehörigen Abtriebskolonne 9, wird bei einem Druck gearbeitet, der beträchtlich unter dem Betriebsdruck der Vordestillationskolonne 1 liegt. Im übrigen wird die Extraktionsstufe nach den bekannten Prinzipien der Extraktionsdestillation betrieben. Das heißt, die Nichtaromaten des Einsatzproduktes werden über die Leitung 19 über Kopf aus der Extraktivdestillationskolonne 12 abgezogen, während der aromatenhaltige Extrakt aus dem Sumpf dieser Kolonne über die Leitung 20 entfernt und in die Abtriebskolonne 9 eingeleitet wird. In dieser werden die als Extrakt gewonnenen Aromaten destillativ von dem ebenfalls im Extrakt enthaltenen selktiven Lösungsmittel abgetrennt. Letzteres wird über die Leitung 21 aus dem Sumpf der Abtriebskolonne 9 abgezogen und am Kopf der Extraktivdestillationskolonne 12 wieder aufgegeben, während gleichzeitig die gewonnenen Aromaten die Abtriebskolonne 9 als Kopfprodukt über die Leitung 22 verlassen. Durch die Leitung 23 kann gegebenenfalls frisches Lösungsmittel in den Lösungsmittelkreislauf eingespeist werden.

Das Sumpfprodukt aus der Vordestillationskolonne 1 wird durch die Leitung 24 abgezogen und gelangt über den Wärmeaustauscher 4 in den Sammelbehälter 25, von dem aus es über die Leitung 15 seiner weiteren Verwendung zugeführt wird. Im Wärmeaustauscher 4 wird die im Sumpfprodukt enthaltene Wärme in indirektem Wärmeaustausch auf das Einsatzprodukt in der Leitung 5 übertragen.

Die nachfolgenden Verfahrensbeispiele sollen die Wirksamkeit der erfindungsgemäßen Arbeitsweise unter Beweis stellen.

3

0 073 945

## Beispiel 1

Dieses Beispiel betrifft die Gewinnung von Reinbenzol aus einem druckraffinierten Kokereirohbenzol, wobei das Einsatzprodukt gemäß dem Fließ-Schema zunächst einer Vordestillation unterworfen wird und das dabei anfallende Kopfprodukt in einer Extraktivdestillation mit N-Formylmorpholin als selektivem Lösungsmittel weiterbehandelt wird.

Das Einsatzprodukt hat hierbei folgende Zusammensetzung:

| | |
|---|---|
| C 5 — KW | 0,04 Gew.-% |
| C 6 — KW | 0,78 Gew.-% |
| Methylcyclopentan | 0,20 Gew.-% |
| iso C 7 — KW | 0,12 Gew.-% |
| Cyclohexan | 0,35 Gew.-% |
| nC 7 + Dimethylcyclopentan | 0,16 Gew.-% |
| Methylcyclohexan | 0,25 Gew.-% |
| Benzol | 75,30 Gew.-% |
| Paraffinisch C 8 — KW | 0,07 Gew.-% |
| Dimethylcyclohexan | 0,48 Gew.-% |
| Toluol | 20,05 Gew.-% |
| C 8 — Aromaten | 2,20 Gew.-% |

Im Teil A des Versuches wird nach dem Stand der Technik gearbeitet. Das heißt, das Einsatzprodukt wird einer Vordestillation bei normalem Druck (Druck am Kolonnenkopf maximal 1,1 bar) unterworfen, wobei das anfallende Kopfprodukt in die nachgeschaltete Extrativdestillationskolonne eingeleitet wird, die mit einem Druck von 2 bar betrieben wird. Ein Wärmeverbund im Sinne der vorliegenden Erfindung findet in diesem Falle zwischen der Vordestillation und der Extraktionsstufe nicht statt. Das als Kopfprodukt der Abtriebskolonne gewonnene Reinbenzol enthält 100 ppm Toluol und 350 ppm Nichtaromaten als Verunreinigungen. Die Benzolausbeute beträgt hierbei 99,8%. Der Gesamtwärmebedarf des Verfahrens beträgt hierbei:

| | |
|---|---|
| Vordestillation | 1440 kJ/kg Benzol |
| Extraktionsstufe | 920 kJ/kg Benzol |
| Gesamtwärmebedarf | 2360 kJ/kg Benzol |

In Teil B des Versuches wurde dagegen bei gleichem Einsatzprodukt die Vordestillation unter folgenden Bedingungen durchgeführt:

| | |
|---|---|
| Druck am Kolonnenkopf | 17 bar |
| Kopftemperatur | 209° C |
| Sumpftemperatur | 281° C |
| Rückflußverhältnis | 1 : 3,2 |

Die Arbeitsbedingungen in der Extraktionsstufe (Extraktivdestillation + Abtrieb) wurde gegenüber Teil A nur insofern geringfügig verändert, daß wegen des höheren Methylcyclohexangehaltes des Kopfproduktes der Vordestillation der Anteil des in der Extraktivdestillation verwendeten Lösungsmittel geringfügig erhöht wurde. Reinheit und Ausbeute des gewonnenen Benzols entsprechen dabei praktisch den Ergebnissen in Teil A. Erfindungsgemäß werden jedoch die Kopfproduktdämpfe der Vordestillation zur Kolonnenbeheizung in der Extraktionsstufe herangezogen, wobei sich für den Wärmebedarf des Verfahrens folgende Situation ergibt:

| | |
|---|---|
| Vordestillation | 1270 kJ/kg Benzol |
| Extraktionsstufe | 1230 kJ/kg Benzol |

Im Vergleich zur Vordestillation bei Normaldruck (Teil A) ist der Wärmebedarf der Vordestillation bei erhöhtem Druck (Teil B) trotz eines etwas höheren Rückflußverhältnisses niedriger, weil bei dem angewandten Druck die Verdampfungswärme um ca. 30% niedriger ist. Bei der erfindungsgemäßen Arbeitsweise ist es außerdem nicht erforderlich, den Wärmebedarf der Extraktionsstufe in Form gesonderter Energie (vorzugsweise Dampf) aufzubringen. Dieser Wärmebedarf wird vielmehr vollständig durch den Wärmeinhalt der Kopfproduktdämpfe der Vordestillation gedeckt. Die Kopfproduktdampftemperatur von 209° C ist hierbei völlig ausreichend. Tatsächlich sind normalerweise für die Beheizung der Extraktionsstufe nur 80—90% der Kopfproduktdampfmenge erforderlich, so daß der Rest für die Vorwärmung des Einsatzproduktes genutzt werden kann. Der Gesamtwärmebedarf für Teil B ist deshalb insgesamt nur mit 1270 kJ/kg Benzol anzusetzen.

4

0 073 945

Ein Vergleich von Teil A mit Teil B ergibt deshalb, daß für Teil B nur

$$\frac{1270 \cdot 100}{2360} = 54\%$$

des Wärmebedarfs von Teil A erforderlich sind. Die erzielte Energieeinsparung beträgt somit 46%.

Beispiel 2

Dieses Beispiel betrifft die Gewinnung von Reinbenzol aus einem vollhydrierten Pyrolysebenzin. Das Einsatzprodukt hat dabei folgende Zusammensetzung:

| | |
|---|---|
| C 5 — KW | 0,06 Gew.-% |
| C 6 — KW | 1,23 Gew.-% |
| Methylcyclopentan | 4,73 Gew.-% |
| iso C 7 — KW | 1,97 Gew.-% |
| Cyclohexan | 2,05 Gew.-% |
| n C 7 + Dimethylcyclopentan | 2,59 Gew.-% |
| Methylcyclohexan | 1,05 Gew.-% |
| Benzol | 60,20 Gew.-% |
| Paraffinische C 8 — KW | 0,99 Gew.-% |
| Dimethylcaclohexan | 0,96 Gew.-% |
| Toluol | 23,97 Gew.-% |
| C 8 — Aromaten | 0,20 Gew.-% |

Die Versuchsdurchführung entspricht hierbei prinzipiell der im Beispiel 1 gewählten Arbeitsweise. Für Teil A (Vordestillation unter Normaldruck) ergibt sich hierbei folgender Wärmebedarf:

| | |
|---|---|
| Vordestillation | 1290 kJ/kg Benzol |
| Extraktionsstufe | 1000 kJ/kg Benzol |
| Gesamtwärmebedarf | 2290 kJ/kg Benzol |

Das gewonnene Reinbenzol enthält dabei noch 100 ppm Toluol und 400 ppm Nichtaromaten als Verunreinigungen.

In Teil B wird die Vordestillation dagegen unter folgenden Bedingungen durchgeführt:

| | |
|---|---|
| Druck am Kolonnenkopf | 17 bar |
| Kopftemperatur | 209° C |
| Sumpftemperatur | 252° C |
| Rückflußverhältnis | 1 : 3,6 |

Für den Wärmebedarf ergeben sich dabei folgende Werte:

| | |
|---|---|
| Vordestillation | 1420 kJ/kg Benzol |
| Extraktionsstufe | 1290 kJ/kg Benzol |

Da jedoch auch hier der Wärmebedarf der Extraktionsstufe vollständig durch den Wärmeinhalt der Kopfproduktdämpfe der Vordestillation gedeckt wird, ergibt sich für den Wärmebedarf von Teil B gegenüber Teil A folgende Rechnung:

$$\frac{1420 \cdot 100}{2290} = 62\%$$

Die erzielte Energieeinsparung beträgt somit 38%.

Die vorliegenden Beispiele belegen die Wirksamkeit des erfindungsgemäßen Verfahrens und zeigen, daß damit bei gleicher Reinheit und Ausbeute der gewonnenen Aromaten ganz beträchtliche Energieeinsparungen erzielt werden können.

**Patentansprüche**

1. Verfahren zur Gewinnung reiner Aromaten aus Kohlenstoffgemischen, die neben diesen Aromaten einen beliebig hohen Gehalt an Nichtaromaten enthalten, durch Extraktivdestillation mit N-substituierten Morpholinen als selektivem Lösungsmittel, wobei der Extraktionsstufe eine Vordestillation zugeordnet ist, dadurch gekennzeichnet, daß die Vordestillation bei erhöhtem Druck bis zu 20 bar und bei Temperaturen bis zu 300° C durchgeführt wird, wobei der Druck jeweils so eingestellt wird, daß die Vordestillation bei höherer Temperatur betrieben werden kann als die Extraktionsstufe und der Wärmeinhalt der dabei anfallenden Dämpfe zur Kolonnenbeheizung in der bei niedriger Temperatur betriebenen Extraktionsstufe genutzt wird.

2. Verfahren nach Anspruch 1, dadurch gekennzeichnet, daß die in der Vordestillation anfallenden Dämpfe unter vollständiger Kondensation zur Kolonnenbeheizung in der Extraktionsstufe genutzt werden und das dabei anfallende Kondensat als Einsatzprodukt in die Extraktionsstufe eingeleitet wird.

3. Verfahren nach den Ansprüchen 1 und 2, dadurch gekennzeichnet, daß ein Teil der in der Vordestillation anfallenden Dämpfe zur Vorwärmung des Einsatzproduktes genutzt wird.

4. Verfahren nach den Ansprüchen 1 bis 3, dadurch gekennzeichnet, daß für die Vordestillation das Rückflußverhältnis und die Bodenzahl der Kolonne so gewählt werden, daß die dabei am Kopf der Kolonne anfallende Dampfmenge für die Kolonnenbeheizung in der Extraktionsstufe sowie die gegebenenfalls erforderliche Vorwärmung des Einsatzproduktes ausreicht.

5. Verfahren nach den Ansprüchen 1 bis 4, dadurch gekennzeichnet, daß die Extraktionsstufe unter einem Druck von 1 bis 3 bar betrieben wird.

6. Verfahren nach den Ansprüchen 1 bis 5, dadurch gekennzeichnet, daß in der Extraktionsstufe N-Formylmorpholin als selektives Lösungsmittel verwendet wird.

## Claims

1. Process for the production of pure aromatics from hydrocarbon mixtures which, in addition to these aromatics, contain any possible quantity of non-aromatics, by extractive distillation with N-substituted morpholines as the selective solvent, the extraction stage being associated with an initial distillation, characterised in that the initial distillation is carried out under an elevated pressure of up to 20 bars and at temperatures of up to 300° C, the pressure being set in each case such that the initial distillation can be operated at a higher temperatures than the extraction stage and the heat content of the vapours thus obtained is utilised for column heating in the extraction stage operated at a low temperature.

2. Process according to Claim 1, characterised in that the vapours obtained in the initial distillation being utilised, with complete condensation for column heating in the extraction stage and the condensate thus obtained being introduced as the feed product into the extraction stage.

3. Process according to Claims 1 and 2, characterised in that a part of the vapours obtained in the initial distillation is utilised for preheating the feed product.

4. Process according to Claims 1 to 3, characterised in that the reflux ratio and the number of plates in the column for the initial distillation are selected such that the quantity of vapours thus obtained at the top of the column is sufficient for the column heating in the extraction stage and for the preheating of the feed product, which may be necessary.

5. Process according to Claims 1 to 4, characterised in that the extraction stage is operated under a pressure from 1 to 3 bars.

6. Process according to Claims 1 to 5, characterised in that N-formylmorpholine is used as the selective solvent in the extraction stage.

## Revendications

1. Procédé d'isolement d'hydrocarbures aromatiques purs à partir de mélanges d'hydrocarbures qui contiennent, outre ces hydrocarbures aromatiques, des hydrocarbures non aromatiques en teneur arbitrairement élevée, par distillation extractive au moyen de morpholines N-substituées comme solvant sélectif, dans lequel une prédistillation est associée à un stade d'extraction, caractérisé en ce que la prédistillation est exécutée sous une pression accrue jusqu'à 20 bars et à des températures jusqu'à 300° C, tandis que la pression est toujours choisie de façon que la prédistillation puisse être exécutée à une température plus élevée que le stade d'extraction et que la capacité calorifique des vapeurs ainsi obtenues soit utilisée au chauffage des colonnes au stade d'extraction exécuté à une température plus basse.

2. Procédé suivant la revendication 1, caractérisé en ce que les vapeurs obtenues à la prédistillation sont utilisées sous condensation complète pour le chauffage des colonnes au stade d'extraction et le condensat ainsi obtenu est amené comme matière première au stade d'extraction.

3. Procédé suivant les revendications 1 et 2, caractérisé en ce qu'une fraction des vapeurs obtenues à la prédistillation est utilisée pour le préchauffage de la matière première.

4. Procédé suivant les revendications 1 à 3, caractérisé en ce que le taux de reflux pour la prédistillation et le nombre de plateaux de la colonne sont choisis de manière que la quantité de vapeur obtenue en tête de colonne soit suffisante pour le chauffage des colonnes au stade d'extraction ainsi que pour

**0 073 945**

le préchauffage éventuellement nécessaire de la matière première.

5. Procédé suivant les revendications 1 à 4, caractérisé en ce que le stade d'extraction est exécuté sous une pression de 1 à 3 bars.

6. Procédé suivant les revendications 1 à 5, caractérisé en ce que le stade d'extraction est exécuté au moyen de N-formylmorpholine comme solvant sélectif.

7

0 073 945